# EUROPEAN PATENT SPECIFICATION

(11) **EP 0 755 235 B1**
(45) Date of publication and mention of the grant of the patent: **17.03.1999**
(21) Application number: 95915626.6
(22) Date of filing: 10.04.1995
(51) Int. Cl.: A61F 13/15, A61F 13/58

(54) **ABSORBENT ARTICLE HAVING FLAPS**
FLÜGEL FÜR SAUGFÄHIGE PRODUKTE
ARTICLE ABSORBANT COMPORTANT DES RABATS

(30) Priority: 15.04.1994 US 228337
(43) Date of publication of application: 29.01.1997
(73) Proprietor: THE PROCTER & GAMBLE COMPANY, Cincinnati, Ohio 45202 (US)
(72) Inventor: RANDALL, Catherine, Jean, Cincinnati, OH 45215 (US); LAVASH, Bruce, William, West Chester, OH 45069 (US); AMOS, Charles, William, Jr., Cincinnati, OH 45231 (US); MAINGOT, Alan, Lawrence, Cincinnati, OH 45247 (US); HERSHBERGER, Michael, Nyle, Cincinnati, OH 45242 (US); BEESON, Jon, Robert, Cincinnati, OH 45215 (US); LOKAR, Stoyan, Cincinnati, OH 45241 (US)
(74) Representative: Hirsch, Uwe Thomas
(86) International application number: US9504389
(87) International publication number: WO9528137

(56) References cited:
- EP-A- 0 331 018
- WO-A-93/06805
- WO-A-95/03025
- WO-A-95/08311

## Description

### FIELD OF THE INVENTION

The present invention relates to absorbent articles such as sanitary napkins, panty liners, adult incontinence devices, and the like. Still more particularly, the present invention concerns absorbent articles having flaps and zones of differential extensibility for relieving the stresses that develop in the flaps when the flaps are folded down and under a wearer's undergarment.

### BACKGROUND OF THE INVENTION

All manner and variety of absorbent articles configured for the absorption of body fluids such as menses, urine, and feces are, of course, well known. Absorbent articles, particularly sanitary napkins, having wings or flaps are disclosed in the literature and are available in the marketplace.

Generally, the flaps extend laterally from a central absorbent means and are intended to be folded around the edges of the wearer's panties in the crotch region. Thus, the flaps are disposed between the edges of the wearer's panties in the crotch region and the wearer's thighs. Commonly, the flaps are provided with an attachment means for affixing the flaps to the underside of the wearer's panties.

The flaps serve at least two purposes. First, the flaps prevent exudates which otherwise would soil the edges of the wearer's panties from doing such. Second, the flaps help stabilize the napkin from shifting out of place, especially when the flaps are affixed to the underside of the panties.

Sanitary napkins having flaps of the various types are disclosed in U.S. Patent 4,687,478, entitled "Shaped Sanitary Napkin With Flaps", which issued to Van Tilburg on August 18, 1987, U.S. Patent 4,608,047, entitled "Sanitary Napkin Attachment Means", which issued to Mattingly on August 26, 1986, U.S. Patent 4,589,876, entitled "Sanitary Napkin", which issued to Van Tilburg on May 20, 1986, U.S. Patent 4,285,343, entitled "Sanitary Napkin", which issued to McNair on August 25, 1981, U.S. Patent 3,397,697, entitled "Disposable Sanitary Shield For Undergarments", which issued to Rickard on August 20, 1968, and U.S. Patent 2,787,271, entitled "Sanitary Napkin", which issued to Clark on April 2, 1957.

While sanitary napkins having flaps are commonly viewed as providing better protection against soiling as compared to sanitary napkins without flaps, the flapped napkins commonly experience problems that keep them from being optimally effective. These problems generally result from the stresses exerted on such flaps when the sanitary napkins are worn.

When the flaps are folded down along the edges of the wearer's panties, stresses are created in the flaps. The stresses are especially high along the fold line at the edges of the wearer's panties where the flaps are bent from the body side of the panty to the underside of the panty. These stresses are caused by fitting a flap around the curved outline of a panty crotch. These stresses are magnified when a wearer sits or crouches because the edges of the panties are pulled outward against the flaps thus increasing the forces against this fold line. When the stresses become too high, the flaps may become detached from the panty and some portion of the aforementioned benefits of the flaps may be lost. In addition, even if the stresses are not sufficient to detach the flaps, they may still be sufficient to cause the flaps to bunch longitudinally inward. This effectively reduces the size of the flaps and the area of the wearer's undergarments that the flaps are able to cover. Thus, there is a commercial need for a way of eliminating or at least reducing the stresses that develop in the flaps when folded, so as to prevent them from becoming detached from the wearer's panties and losing ability to cover a given area of the panties.

A number of variations on the types of flaps described above have been presented in an attempt to solve various problems. U.S. Patent 4,900,320 issued to McCoy on February 13, 1990, discloses a sanitary napkin having flaps affixed at points inward from the longitudinal edge of the napkin. U.S. Patent 4,911,701 issued to Mavinkurve on March 27, 1990, discloses a sanitary napkin having elastic means for providing greater convex shape to the body-facing portion of the central absorbent and for enabling adhesive-free placement of the flaps of the napkin. U.S. Patent 4,940,462 issued to Salerno on July 10, 1990, discloses a sanitary napkin with longitudinally expandable flaps, a concept previously illustrated in Figure 5 of U.S. Patent 4,589,876 issued to Van Tilburg. A sanitary napkin having flaps with stress relief means in the form of notches or slits in particular locations of the sanitary napkin is described in U.S. Patent 4,917,697 which issued to Osborn, III, et al. on April 17, 1990. Although this latter sanitary napkin works quite well, the search for sanitary napkins having improved flaps has continued.

A sanitary napkin comprising the features appearing in the preamble of claim 1 is known from EP-A-0 331 018. In the known sanitary napkin, the flaps have an elasticated portion and the common point from which the corrugations originate lies outside the transverse centerline of each flap.

The stresses described above can also unduly limit the size of the flaps used with an absorbent article since the stresses are typically greater in products having large flaps (that is flaps having a relatively large longitudinal dimension). There is, thus, also a need for an improved stress relief means for relieving the stresses that develop in the flaps, that does not limit the size of the flaps that can be used.

Therefore, it is an object of the present invention to provide an absorbent article, such as a sanitary napkin, having flaps and zones of differential extensibility for relieving the stresses that develop in the flaps when they are folded down along the edges of the crotch of the wearer's undergarments and affixed to the underside of the undergarments.

It is an additional object of the present invention to provide an absorbent article having flaps and zones of differential extensibility that allow larger flaps to be used on the absorbent article, than those of prior products.

These and other objects of the present invention will be more readily apparent when considered in reference to the following description and when taken in conjunction with the accompanying drawings.

### SUMMARY OF THE INVENTION

In accordance with the present invention, an absorbent article, such as a sanitary napkin, having flaps and zones of differential extensibility for relieving the stresses that develop in the flaps when the flaps are folded down along the edges of the wearer's panties in the crotch, is provided.

The sanitary napkin has a principal longitudinal centerline and a principal transverse centerline. The sanitary napkin comprises a main body portion and a pair of flaps associated with the main body portion. The main body portion of the sanitary napkin comprises a liquid pervious topsheet, a liquid impervious backsheet joined to the topsheet, and an absorbent core positioned between the topsheet and the backsheet. The main body portion has two spaced apart longitudinal edges and two spaced apart transverse edges.

The flaps extend laterally outward from the main body portion. The flaps are associated with the main body portion at a juncture along the longitudinal edges of the main body portion. The flaps are divided into a front half and a back half by a flap transverse centerline. The absorbent article has two corner regions located adjacent the area of the ends of the junctures. One corner region is located adjacent the area of the juncture in each direction remote from the principal transverse centerline. The sanitary napkin comprises zones of differential extensibility which allow the corner regions to extend transversely outward to a greater degree than the portions of the sanitary napkin located along the flap transverse centerline. The zones of differential extensibility provide a means for the relief of stresses in the flaps of the sanitary napkin when the sanitary napkin is placed in the wearer's undergarments. A nonlimiting number of alternative types of zones of differential extensibility are disclosed.

### BRIEF DESCRIPTION OF THE DRAWINGS

Figure 1 is a top plan view of a preferred sanitary napkin embodiment of the present invention.
Figure 1A is a lateral cross-sectional view taken along line 1A-1A of Figure 1 through the corner region of one of the flaps of the sanitary napkin.
Figure 1B is a lateral cross-sectional view taken along line 1B-1B of Figure 1 through the center portion of one of the flaps.
Figure 2 is a perspective view of the crotch portion of a women's panties.
Figure 3 is the same perspective view of the women's panties shown in Figure 2 with the sanitary napkin embodiment of Figure 1 being placed therein for use.
Figure 4 is a simplified top plan view of a flap of a sanitary napkin having ring rolled zones of differential extensibility disposed in a radial pattern.

### DETAILED DESCRIPTION OF PREFERRED EMBODIMENTS

The present invention relates to absorbent articles, such as sanitary napkins, adult incontinence devices, panty liners, and the like that are provided with side flaps. More particularly, the present invention relates to absorbent articles having flaps and zones of differential extensibility for relieving the stresses that develop in the flaps when they are folded around and under the edges of the crotch of a wearer's undergarments.

The term "absorbent article", as used herein, refers to articles which absorb and contain body exudates. More specifically, the term refers to articles which are placed against or in proximity to the body of the wearer to absorb and contain the various exudates discharged from the body. The term "absorbent article" is intended to include sanitary napkins, panty liners, and incontinence pads (and other articles worn in the crotch region of a garment). The term "disposable" refers to articles which are intended to be discarded after a single use and preferably recycled, composted, or otherwise disposed of in an environmentally compatible manner. (That is, they are not intended to be laundered or otherwise restored or reused as an absorbent article.) In the preferred embodiment illustrated, the absorbent article is a sanitary napkin designated 20.

The term "sanitary napkin", as used herein, refers to an article which is worn by females adjacent to the pudendal region that is intended to absorb and contain the various exudates which are discharged from the body (e.g., blood, menses, and urine). The present invention, however, is not limited to the particular types or configurations of absorbent articles shown in the drawings.

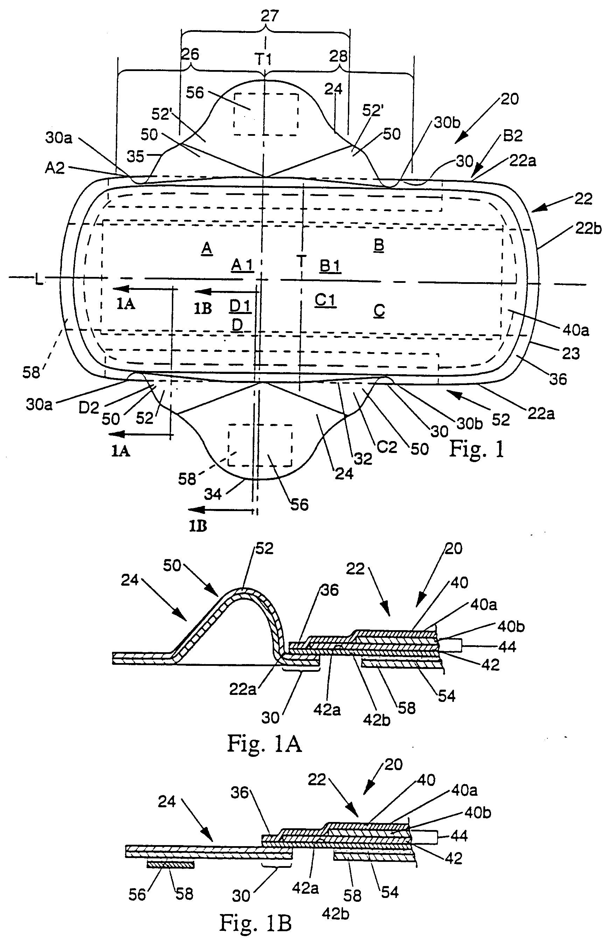

A representative embodiment of a sanitary napkin 20 having flaps and zones of differential extensibility is shown in Figure 1. As shown in Figure 1, the sanitary napkin 20 basically comprises an absorbent means represented by central absorbent pad (or "main body portion") 22, and two flaps 24. (In the discussion that follows, unless otherwise noted, the sanitary napkin described herein will have two flaps. While it is not necessary that the napkin have two flaps, two flaps are preferred over one flap. Also, while it is not necessary that the flaps be mirror images of one another, they preferably are. Thus, the description of one flap will be a description of the other, and, for clarity, discussion of the second flap may be omitted.)

The sanitary napkin 20 has two centerlines, a principal longitudinal centerline l and a principal transverse centerline t. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

The sanitary napkin 20 is comprised of a topsheet 40, a backsheet 42, and an absorbent core 44. At least a part of the topsheet 40, backsheet 42, and absorbent core 44 comprise the main body portion 22. The basic components of the main body portion 22 of the sanitary napkin 20, and the assembly of the same, can comprise any of those that are known in the art. Some particularly preferred sanitary napkins are described in greater detail in U.S. Patent 4,917,697 issued to Osborn, U.S. Patent 5,009,653 issued to Osborn, and U.S. Patent 5,281,208 issued to Thompson, et al.

The main body portion 22 is the portion of the sanitary napkin 20 that contains an absorbent means, such as absorbent core 44. The main body portion 22 has a liquid pervious body contacting surface (represented in Figure 1A by topsheet 40) and an opposed liquid impervious surface (represented in Figure 1A by backsheet 42). It is to be understood that the embodiment illustrated is only one possible embodiment, albeit a preferred one. Other possible embodiments include one in which an absorbent core 44 is essentially completely wrapped with a topsheet before it is placed on a backsheet. The main body portion 22 can also comprise an absorbent core which possesses sufficient integrity to stand alone and is liquid pervious on one surface while the other surface has been treated to render it liquid impervious.

The main body portion 22 may be relatively thick or relatively thin. The main body portion 22 may also be relatively narrow. A narrow main body portion 22 may be effective because the overall configuration and use of sanitary napkin 20 results in main body portion 22 being maintained in close proximity to the body. Such proximity of main body portion 22 places it precisely where it should be: very near the body at the vaginal opening. The main body portion 22 can then absorb the vast majority of the menstrual fluid (menses) before it has an opportunity to flow along the sides of the main body portion 22. A thin main body portion may also be desired because it is typically comfortable to the user.

The flaps 24 shown in Figures 1 and 1A are comprised of separate pieces of material which are attached to the main body portion 22. In alternative embodiments, such as those shown in U.S. Patent 4,917,697 issued to Osborn, the flaps 24 may be integral with the main body portion 22. In such a case, the topsheet 40 may form one surface of both the flaps 24 and the main body portion 22, and the backsheet 42 may form the other surface of the same. In addition, the absorbent material of the sanitary napkin 20 may extend into the flaps 24 to form a flap absorbent core, as described in greater detail in U.S. Patent 4,917,697.

The flaps 24 are each associated with main body portion 22 along a juncture. This is typically a longitudinally-oriented (or "longitudinal") juncture, such as lines of juncture 30. As used herein, the terms "juncture" (or "line of juncture") refer to regions where the flaps 24 extend from or are joined to the main body portion 22. These regions can be any of various curved or straight lines, but they are not limited to lines. Thus, these regions can comprise flanges, strips, intermittent lines, and the like. In the embodiment illustrated in Figure 1, line of juncture 30 is a relatively straight line.

It is not necessary that the flaps 24 extend from (or be joined along) the longitudinal edges 22a of the main body portion 22. The flaps 24 can joined inward (or "inboard") from the longitudinal edges 22a toward the longitudinal centerline. The flaps 24 can, thus, each be joined to the main body portion 22 along the principal longitudinal centerline l, or along the longitudinal edges 22a of the main body portion 22, or at any place between the principal longitudinal centerline l and the longitudinal edges 22a of the main body portion 22. The flaps 24 will, of course, generally be on opposite sides of the principal longitudinal centerline l.

The flaps 24 have a proximal edge 32 adjacent the line of juncture. A distal edge (or "free end") 34 is remote from the line of juncture 30. The desired characteristics of the flaps 24 of the sanitary napkin 20 are described in greater detail in U.S. Patent 4,589,876, entitled "Sanitary Napkin", which issued to Van Tilburg on May 20, 1986, U.S. Patent 4,687,478, entitled "Shaped Sanitary Napkin With Flaps", which issued to Van Tilburg on August 18, 1987, and in the originally-filed patent applications directed to absorbent articles having flaps and zones of differential extensibility discussed below. As shown in Figure 1, each flap 24 is divided into a front half 26, and a back half 28 by a flap transverse centerline t₁. The flap transverse centerline t₁ may coincide with the principal transverse centerline t of the sanitary napkin, but this is not absolutely required. The flap transverse centerline t₁ extends through the principal longitudinal centerline l to divide the sanitary napkin into four quarters A, B, C, and D.

The quarters A, B. C, and D each comprise a first portion or zone (such as A₁, B₁, C₁, and D₁) adjacent at least a portion of the principal longitudinal centerline l and the flap transverse centerline t₁. A second portion or zone (A₂B₂, C₂, and D₂) is outboard of and complementary with the first portion. (The terms "outboard" or "outward", as used herein, mean generally spaced in a direction away from these centerlines. The term "complementary", as used herein, means that the first and second portions form an entire quarter.)

The sanitary napkin 20 has at least one zone of differential extensibility (or "zone of extensibility", or simply "zone") 50. Preferably, as shown in Figure 1, the sanitary napkin 20 has at least four zones of differential extensibility 50, one in each quarter of the sanitary napkin 20. The zones of differential extensibility 50 relieve the stresses which develop in the flaps 24 when they are folded around a panty crotch. Since the zones of differential extensibility 50 relieve stresses in the flaps, they may be referred to herein as a type of "stress relief means".

The term "zone of differential extensibility", as used herein, refers to a portion of the sanitary napkin 20 which is capable of extending a differing amount (preferably a greater amount), than surrounding portions of the sanitary napkin 20. These "surrounding portions" of the sanitary napkin comprise the first portions of the quarters. The zones of differential extensibility 50, thus, comprise the second portions of each quarter.

The zones of differential extensibility 50, can also be described as being located at least in the corner regions 52 of the sanitary napkin 20. (Thus, the second portions A₂, B₂, C₂, and D₂ of the quarters preferably comprise the corner regions 52 of the sanitary napkin 20.) The sanitary napkin 20 preferably has four corner regions 52 (two by each flap, and one in each quarter). The term "corner regions" 52, as used herein, refers to portions of the sanitary napkin 20 that are generally located along or adjacent a portion of the longitudinal juncture of each flap 24. The corner regions 52 for each flap 24 are located in two areas in the regions of the ends 30a and 30b of each juncture 30. One corner region 52 is located adjacent the longitudinal juncture 30 in the front half 26 of the flap 24. The other is adjacent the longitudinal juncture 30 in the back half 28 of the flap 24. The corner regions 52 are preferably at least partially disposed longitudinally away from the flap transverse centerline t₁ in each direction. (Thus, the corner regions 52 may be described as being longitudinally "remote" from the flap transverse centerline t₁ or the center portion 27 of the flaps.)

In the most preferred case, the zones of differential extensibility 50 are located along a portion of the fold line where the flaps 24 are folded around the wearer's panty crotch. The fold line will typically be located along or adjacent the longitudinal juncture 30 of each flap 24. Since the terms "portions", "zones", and "regions", as used herein, refer to general areas, the zones of differential extensibility 50 and the corner regions 52 are, thus, not limited to points which lie precisely on the lines of juncture 30. Typically, they will include both those points which lie on the lines of juncture 30 as well as the surrounding areas of the sanitary napkin 20 (which include the aforementioned fold lines). The longitudinal junctures, thus, typically serve as good approximations for the location of the zones of differential extensibility 50.

The corner regions 52 are designated as such because they typically include the "corners" formed along the periphery 23 of the sanitary napkin 20. The "corners" occur where the edges 35 of the flaps 24 intersect with the longitudinal side edges 22a of the main body portion 22 when the sanitary napkin 20 is shown in a plan view. It is not necessary for there to be a sharp angle formed at the intersection of these edges, or for lines of demarcation to designate the same, however. (Another way to describe the corner regions 52 is with reference to U.S. Patent 4,917,697 issued to Osborn, III, et al. The corner regions 52 described herein are typically comprised at least of those areas shown as having slits or notches in the Osborn, et al. patent. (For simplicity, these areas may be referred to herein as "notch regions"). However, the corner regions 52 in the present invention preferably encompass a larger area than the slits or notches shown in the Osborn patent.) The portions of the flaps 24 in the corner regions 52 of the sanitary napkin 20 may be referred to as the "corner regions of the flaps" or "flap corner regions". These may be separately designated 52' although they are still considered to comprise the corner regions 52, per se.

The zones of differential extensibility 50 are preferably primarily extensible in a greater amount generally outward in the transverse direction. This is generally in the direction of the arrows shown in Figure 1. As used herein, the phrase "generally in the transverse direction" means that the extensibility has a transverse component. All of the extension, however, need not be exactly parallel to the principal transverse centerline of the sanitary napkin. The extensibility is preferably oriented more in the transverse direction than in the longitudinal direction. In addition, it is also possible for the alternative types of zones of differential extensibility described herein to be provided having a component of extensibility in any other direction, or in more than one direction.

The zone(s) of differential extensibility 50 comprise a structure that preferably is capable of extending a greater amount generally in the transverse direction than the surrounding portions of the sanitary napkin. The differential extensibility referred to herein, however, must be elasticless. That is, it must be accomplished without the use of separate elastic pieces, strands, or materials to contract one or more portions of the sanitary napkin. The zones of differential extensibility must also be accomplished without leaving uncovered slits or notches in portions of the sanitary napkin that cover the wearer's undergarments. This will have the advantage that exudates will not be able to travel through the slits or notches to soil the wearers undergarments.

Suitable structures for the zones of differential extensibility 50 include, but are not limited to zones of material that are mechanically strained, corrugated, "ring rolled", folded, pleated, or joined along a curved juncture. These structures (although sometimes shown only as being part of the flaps 24), can comprise portions of the main body portion 22, portions of the flaps 24, or both. They can be integral parts of these components of the sanitary napkin, or separate elements, such as pieces of material, joined to the sanitary napkin. Absorbent articles that are provided with a number of types of zones of differential extensibility are described in the originally-filed patent applications directed to absorbent articles having flaps and zones of differential extensibility, allowed U.S. Patent No. 5,389,094 entitled "Absorbent Article Having Flaps and Zones of Differential Extensibility" issued February 14, 1995, in the name of Lavash, et al., allowed U.S. Patent No. 5,354,400 entitled "Method of Making Absorbent Article Having Flaps and Zones of Differential Extensibility" issued October 11, 1994, and allowed U.S. Patent No. 5,344,416 entitled "Absorbent Article Having Inwardly-Folded Pleated Flaps" issued September 6, 1994, in the name of Niihara, et al. (which are all combined in PCT Publication No. WO 93/06805 published April 15, 1993). The present application is directed to several alternative variations of such absorbent articles.

Figures 1 and 1A also show the fasteners, such as adhesive attachment means, central pad adhesive 54 and flap adhesive 56, which are adapted to secure the sanitary napkin 20 to the crotch region of an undergarment. The central pad adhesive 54 provides an adhesive attachment means for securing main body portion 22 in the crotch portion of a panty. The outer surface of flap 24, adjacent the distal edge 34 of the flap, is preferably coated with a flap adhesive 56. The flap adhesive 56 is used to assist in maintaining the flap 24 in position after it is wrapped around the edge of the crotch portion of the panty as described below. The flaps 24 can be maintained in position by attaching the flaps 24 to the undergarment, or to the opposing flap. Suitable adhesive fasteners are described in greater detail in U.S. Patent 4,917,697.

The fasteners used with the present invention are not limited to adhesive attachment means. Any type of fastener used in the art can be used for this purpose. For example, the sanitary napkin 20 could be secured to the wearer's undergarment by mechanical fasteners and by other types of fasteners such as the fastener described in U.S. Patent 4,946,527 entitled "Pressure-Sensitive Adhesive Fastener and Method of Making the Same" issued to Battrell on August 7, 1990. For simplicity, however, the fasteners will be described in terms of adhesive attachment means.

The adhesive attachment means are respectively covered by removable release liners, central pad release liner and flap release liner, both designated 58. The pressure-sensitive adhesives should be covered with release liners 58 to keep the adhesives from sticking to extraneous surfaces prior to use. Suitable release liners are described in U.S. Patent 4,917,697. In particularly preferred embodiments, the central pad adhesive 54 can be covered by a releasable wrapper such as that described in U.S Patent 4,556,146 issued to Swanson, that also serves as an individual package for the sanitary napkin.

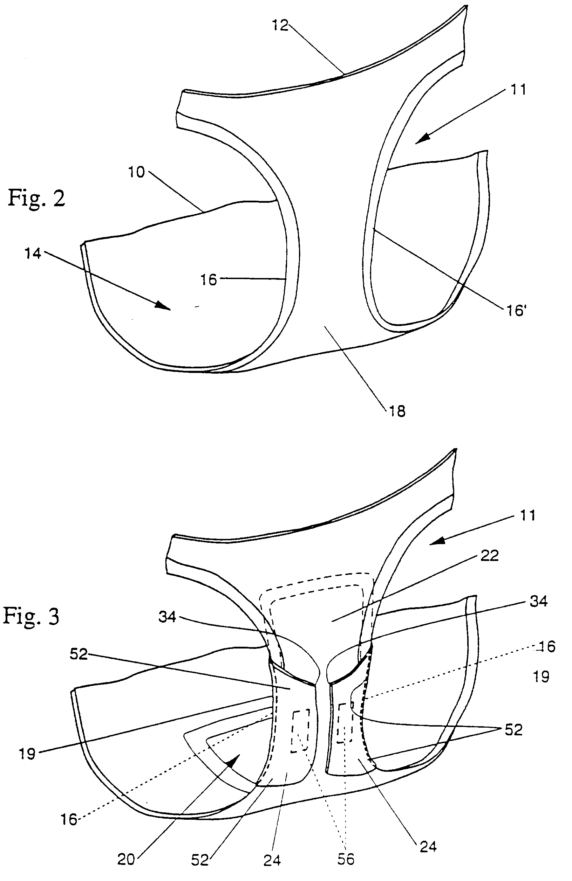

The sanitary napkin of the present invention will now be described in greater detail with relation to the function of the same in the wearer's undergarments. Figure 2 is a depiction of the crotch portion 14 of an undergarment 11 of the type commonly worn by many women and well known as a panty. A panty 11 comprises a front section 10, a back section 12, and a crotch portion 14 which joins the front and back sections. The crotch portion 14 comprises two side edges 16 and center crotch portion 18.

The sanitary napkin 20 of the present invention is utilized by removing the release liners 58 and placing the sanitary napkin 20 in a panty 11 as shown in Figure 3. The center of main body portion 22 is placed in crotch portion 14 of the panty with one end of main body portion 22 extending towards the front section 10 of the panty and the other end towards the back section 12. The backsheet 42 is placed in contact with the inner surface of center crotch portion 18 of the panty. Central pad adhesive 54 maintains main body portion 22 in position. The distal portions of flaps 24 are folded around the side edges 16 of the panty. The flap adhesives preferably secure the flaps 24 to the underside of the panty.

When the flaps 24 are folded down around the edge 16 of the crotch portion 14 of the panty, stresses are developed in the flaps, particularly in the corner regions 52' of the same. These stresses are magnified when the flaps 24 are folded under the panty and attached to the panty's underside. The stresses are further magnified when the panty is pulled up into position and the elastics in the panty edges 16 force the folded portion of the flaps into the uppermost part of the wearer's crotch and thigh.

The stresses are most highly concentrated along the fold 19 where the flap 24 changes from being disposed on the, body side of the panty to being located on the underside of the panty. In other words, the stresses are concentrated at the edge 16 of the crotch portion 14 of the panty 11. The stresses in the flaps 24 generally follow the arc formed by the edges 16 of the crotch portion 14. Depending on the design of the sanitary napkin, however, the arc of concentrated stresses may or may not exactly coincide with the lines of juncture 30 between the flaps 24 and the main body portion 22. These stresses may cause the corner regions 52' of the flaps 24 to bunch longitudinally inward. This reduces the area of the wearer's undergarments the flaps are able to cover. If the stresses are great enough, the flaps 24 can become detached from the panty and the flaps 24 will be less than optimally effective.

In order to eliminate, or at least reduce these stresses, the sanitary napkin 20 is provided with zones of differential extensibility 50. The zones of differential extensibility 50 preferably reduce the stresses along the fold 19 to such a degree that the flaps 24 will remain attached to the underside of the panty and will not lose their ability to cover a given area of the wearer's undergarments.

Figures 1 and 1A show a sanitary napkin which has one representative type of zones of differential extensibility 50. The type of zones of differential extensibility shown in Figs. 1 and 1A is described as being a "representative" of the various types of zones of differential extensibility described herein because it is a relatively simple version of the concept that can be used for purposes of illustration. The characteristics of the type of zones of differential extensibility 50 shown in Figs. 1 and 1A (as well as several other types of zones of differential extensibility) are described in greater detail in the aforementioned U.S. Patent Nos. 5,354,400, 5,389,094 and 5,344,416.

In the embodiment shown in Figures 1 and 1A, the zones of differential extensibility 50 comprise portions of the sanitary napkin 20 that have slack provided therein. These portions of the sanitary napkin 20 comprise at least the flap corner regions 52'. The slack is provided to the sanitary napkin 20 in the embodiment shown in Figures 1 and 1A by pre-stretching (or "pre-straining") the corner regions of the flaps 52'. This can be accomplished by heating and then stretching the flap corner regions generally in the transverse direction. This heating and stretching increases the size of the flap corner regions 52'. Thus, when the sanitary napkin is laid out as shown in Figures 1 and 1A with the flaps 24 extended, there is excess material in the flap corner regions 52'. This excess material allows the flaps 24 to be folded around the crotch of the wearer's panties without stresses being created in the corner regions 52. In addition to pre-stretching, a number of alternative ways of providing zones of differential extensibility are described in greater detail below in conjunction with the embodiments shown in the remaining drawing figures.

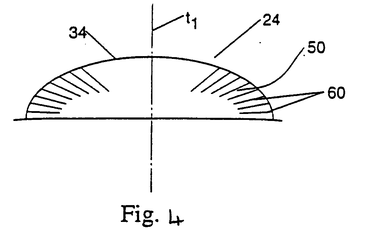

Figure 4 shows a flap 24 having a pattern of ring rolling with radial fold lines 60. The orientation of the fold lines 60 is such that the fold lines 60 on each side of the flap transverse centerline t₁ radiate outward from a single point. This has the advantage of providing greater extensibility at the distal edge 34 of the flap where it is needed most.

The flap shown in Fig. 4 can be made by running the flap material through a set of rolls with meshing teeth, or by using plates with meshing teeth. In either case, the teeth are preferably slightly offset from a perfectly aligned meshing arrangement to provide a better appearing product with regions of higher extensibility and regions of lower extensibility within the zones of differential extensibility. In addition, the zones of differential extensibility can also be provided by performing the "SELFing" process described in WO-A-95/07675 corresponding to USSN 08/124,180 entitled "Absorbent Articles Having Panty Covering Components Comprising Extensible Web Materials Which Exhibit Elastic-Like Behavior" filed by Michele A. Mansfield, et al. on September 17, 1993, and U.S. Patent No. 5,554,145 entitled "Absorbent Article With Multiple Zone Structural Elastic-Like Film Web Extensible Waist Feature" issued to Donald C. Roe, et al. on September 10, 1996 on portions of the absorbent article which may provide elastic-like properties on these portions without the need for attaching separate elastic materials to the absorbent article.

The embodiment shown in Fig. 4 can be manufactured or assembled in several different manners. The zones of differential extensibility can be imparted to the sanitary napkin 20 after the sanitary napkin is completely assembled where the flaps 24 have been previously attached to the main body portion 22. Alternatively, the zones of differential extensibility can be applied to one or more separate components that comprise the flaps 24 and this component (or components) can thereafter be attached to the main body portion 22 to complete the formation of the sanitary napkin 20.

When viewing the various alternative embodiments, it should be understood that the features provided on the absorbent articles described herein are not limited to the particular embodiments shown in the drawings. These features can be combined or modified in any desired manner. Likewise, the techniques described with reference to the assembly of the various embodiments can also be used to make several of the other variations of the present invention.

While the present invention has been described in terms of a sanitary napkin, the flaps and zones of differential extensibility described herein can, as noted above, be provided on other types of absorbent articles such as panty liners and incontinence products. Suitable absorbent articles in the form of pantiliners are disclosed in U.S. Patent 4,738,676 entitled "Pantiliner" issued to Osborn on April 19, 1988. Suitable absorbent articles, at least some of which are in the form of adult incontinence products, are described in U.S. Patent 5,300,054 issued to Barry R. Feist, et al., and U.S. Patent 5,304,151 issued to John R. Noel, et al. The absorbent articles described herein can also be provided with other features such as tucked flaps and the like that are described in U.S. Patent 5,281,209 entitled "Absorbent Article Having Tucked Flaps" issued to Osborn, et al.

A number of patents, patent applications (and any patents which issue thereon, as well as any corresponding published foreign patent applications), and publications are mentioned throughout this description. It is expressly not admitted, however, that any of the documents mentioned herein teach or disclose the present invention.

## Claims

1. An absorbent article (20) having a principal longitudinal centerline (L) and a principal transverse centerline (T), said absorbent article (20) comprising:
a main body portion (22) having a pair of longitudinal edges (22a), said main body portion (22) comprising a liquid pervious topsheet (40), a liquid impervious backsheet (42) joined to said topsheet (40), and an absorbent core (44) positioned between said topsheet (40) and said backsheet (42); and
a pair of flaps (24), each flap (24) being joined to said main body portion (22) and extending laterally outward beyond the longitudinal edges (22a) of said main body portion (22), said flaps (24) having a proximal edge (32) and a distal edge (34), and said flaps (24) having a flap transverse centerline (t₁) wherein said flaps (24) are provided with corrugated sections (50) which have fold lines (60) that extend outward to at least a portion of the distal edges (34) of said flaps (24) and characterized in that
said fold lines (60) a radiate outwards from a single point on said flap transverse centerline (t₁).

## Patentansprüche

1. Ein absorbierender Artikel (20) mit einer Hauptlängsmittellinie L und einer Hauptquermittellinie T, wobei der genannte absorbierende Artikel (20) umfaßt:
einen Hauptkörperabschnitt (22) mit einem Paar Längsrändern (22a), wobei der genannte Hauptkörperabschnitt (22) ein flüssigkeitsdurchlässiges Deckblatt (40), ein flüssigkeitsundurchlässiges Rückenblatt (42), welches mit dem genannten Deckblatt (40) verbunden ist, und einen absorbierenden Kern (44) umfaßt, welcher zwischen dem genannten Deckblatt (40) und dem genannten Rückenblatt (42) positioniert ist; und
ein Paar Lappen (24), wobei jeder Lappen (24) mit dem genannten Hauptkörperabschnitt (22) verbunden ist und sich quer auswärts über die Längsränder (22a) des genannten Hauptkörperabschnitts (22) hinaus erstreckt, wobei die genannten Lappen (24) einen proximalen Rand (32) und einen distalen Rand (34) aufweisen und die genannten Lappen (24) eine Lappenquermittellinie t₁ aufweisen, wobei die genannten Lappen (24) mit geriffelten Teilstücken (50) versehen sind, welche Faltungslinien (60) aufweisen, welche sich auswärts zu mindestens einem Abschnitt der distalen Ränder (34) der genannten Lappen (24) erstrecken, und dadurch gekennzeichnet ist, daß
die genannten Faltungslinien (60) von einem einzigen Punkt an der genannten Lappenquermittellinie t₁ radial strahlenförmig auswärts wegführen.

## Revendications

1. Article absorbant (20) ayant une ligne médiane principale longitudinale (L) et une ligne médiane principale transversale (T), ledit article absorbant (20) comprenant :
une partie de corps principale (22) ayant une paire de bords longitudinaux (220), ladite partie de corps principale (22) comprenant une feuille de dessus perméable aux liquides (40) une feuille de fond imperméable aux liquides (42) réunie à ladite feuille de dessus (40) et une âme absorbante (44) disposée entre ladite feuille de dessus (40) et ladite feuille de fond (42); et
une paire de rabats (24), chaque rabat (24) étant réuni à ladite partie de corps principale (22) et s'étendant latéralement à l'extérieur au delà des bords longitudinaux (22a) de ladite partie de corps principale (22), lesdits rabats (24) ayant un bord proximal (32) et un bord distal (34), et lesdits rabats (24) ayant une ligne médiane transversale de rabat (t₁), dans lequel lesdits rabats (24) sont munis de sections ondulées (50) qui ont des lignes de pliage (60) qui s'étendent vers l'extérieur jusqu'à au moins une partie des bords distaux (34) desdits rabats (24) et
caractérisé en ce que
lesdites lignes de pliage (60) rayonnent vers l'extérieur à partir d'un point unique sur ladite ligne médiane transversale de rabat (t₁).
